# EUROPEAN PATENT SPECIFICATION

(11) **EP 1 553 898 B1**
(45) Date of publication and mention of the grant of the patent: **11.01.2012**
(21) Application number: 03777758.8
(22) Date of filing: 22.10.2003
(51) Int. Cl.: A61F 2/34, A61L 27/36, A61L 27/58

(54) **USE OF SNAP-ON SEMI ANNULAR AUGMENTS TO INHIBIT MULTI-DIRECTIONAL INSTABILITY AFTER TOTAL HIP ARTHROPLASTY**
VERWENDUNG VON HALBRINGFÖRMIGEN VERGRÖSSERUNGEN MIT SCHNAPPVERSCHLUSS FÜR GELENKPFANNEN ZUR VERMEIDUNG VON MULTI-DIREKTIONALEN INSTABILITÄTEN NACH GESAMTEM HÜFTGELENKERSATZ
UTILISATION D'AJOUTS SEMI-ANNULAIRES A PRESSION POUR INHIBER L'INSTABILITE MULTIDIRECTIONNELLE APRES UNE ARTHROPLASTIE TOTALE DE HANCHE

(30) Priority: 23.10.2002 US 420565 P; 02.07.2003 US 612784
(43) Date of publication of application: 20.07.2005
(73) Proprietor: Wasielewski, Ray C., New Albany, OH 43054 (US)
(72) Inventor: Wasielewski, Ray C., New Albany, OH 43054 (US)
(74) Representative: Main, Malcolm Charles
(86) International application number: PCT/US2003/033401
(87) International publication number: WO 2004/037129

(56) References cited:
- DE-A- 4 239 263
- DE-A- 19 716 051

## Description

### BACKGROUND

### Field of the Invention

The present invention is directed to a prosthetic constraining device having snap-on insert augments that provide, at least, initial stability in proximity to mammalian joints in response to a surgical procedure, said augments being formed of biologically reabsorbable material and circumferentially positionable in proximity to the hip joint (actual and/or prosthetic) in portions surrounding the acetabular cup, to provide stability to the joint as the patient's tissue develops. The biologically reabsorbable augments that augment joint stability for a hip prosthesis are absorbed over time in proportion to the development of the patient's natural tissue providing more permanent stability. The invention is also directed to a method for providing at least temporary stability to a prosthetic hip device.

### Background of the Invention

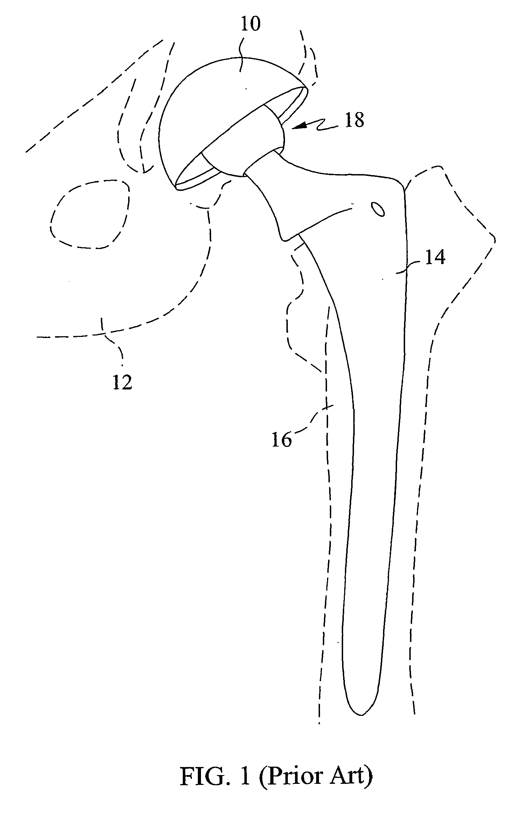

Stability after total hip anthroplasty (THA) is one of the most pressing problems in primary and revision acetabular anthroplasty. FIG. 1 is a drawing of a typical prosthesis utilized in THA, which includes an acetabular cup/shell **10** bonded to the pelvis **12** of a patient and a femoral member **14** or stem bonded to a patient's femur **16,** where the acetabular cup **10** and femoral member **14** are connected together with a ball-joint coupling **18.** If a posterior approach has been done, until the posterior tissues are healed (3 - 6 months), there is dislocation diathesis in this direction. Similarly, there exists an anterior dislocation tendency after anterolateral approach in the hip. In revision THA cases, periarticular capsular releases for exposure can create multidirectional instabilities.

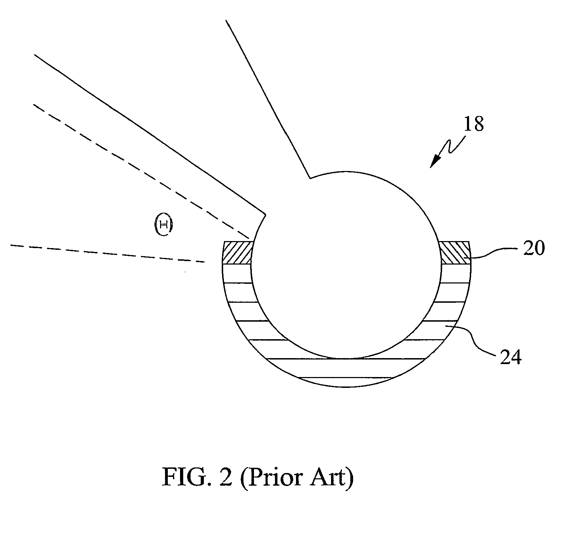

Conventionally, permanent lips and elevations of varying height and length have been added to the periphery of conventional polyethylene inserts (within the acetabular cup) to augment stability. For example, as shown in a cross-sectional view in FIG. 2, prior art techniques to overcome such instability have utilized permanent constraining rings **20** attached to the periphery of the polyethylene insert **24.** However, since the instability is relatively short lived until scar tissue is formed or reformed, permanent elevations and augmented implants such as constraining rings **20** are not always necessary.

These constraining rings **20** may also be the source of impingement and may limit maximal possible range of motion as shown by the angle θ in all 360°. In addition, these constraining rings **20** may not always allow for normal pari-articular scarring to occur to optimize long-term hip stability and range of motion, and may also increase stress transmission to the interfaces of the hip over time resulting in mechanical breakdown of the fixation of the cup to the pelvis or failure of the locking mechanism resulting in backside acetabular wear.

An example of a dislocation guard ring for use on hip endoprotheses which secures to a plastic socket by screws is described in DE19716051. The guard ring itself continues the respective socket curvature to surround the hip ball with the ring being fixed in place on the socket component with screws. The rings and screws are made of a material which can be converted to connective tissue.

### SUMMARY OF THE INVENTION

The invention is defined in the appended claims. Also described is the use of absorbable materials to provide temporary stability after a surgical joint procedure. Exemplary embodiments of the invention provide biologically reabsorbable insert augments mounted to respective hip prosthesis components. Such biologically reabsorbable augments may be absorbed after a time sufficient for patient tissue formation to provide natural constraint and stability to the joint. As these augments are absorbed, any biologic debris would not be considered third body particulate as, for example, poly-L-lactic acid (PLLA) and porcine small intestinal submucosa (SIS) are not harder than any of the prosthesis components. Likewise, the invention has application in any joint reconstruction where the integrity of the constraining tissue has been compromised by injury or as a result of the surgical procedure itself. Further aspects of the present invention are directed toward using biologically reabsorbable materials loaded with agents that may promote tissue formation, fight infection, and promote clotting. The present invention is directed in particular to the subject matter recited in the claims which includes the use of biologically reabsorbable snaps to attach an augment to one of the joint prosthetic components or natural tissue constituents of the joint.

The invention includes semiannular stabilizing augments adapted to be mounted about the acetabular cup assembly of the prosthetic hip joint to provide constraint for the joint and to concurrently provide a range of motion desired by patients after surgery, but with the additional benefit of doing so without substantially increasing the risks of dislocation. At least one exemplary embodiment utilizes a semiannular augment formed from biologically reabsorbable material to temporarily constrain the prosthetic ball within the prosthetic acetabular cup. In such an embodiment, it is desired that the biologically reabsorbable material degrades in general proportion to the level of tissue developed by the patient's own body to supplement constraint of the hip joint. Thus, the artificial constraining augments may degrade inversely proportional to the patient's need for inhibition.

It is a first aspect of the present invention to provide a prosthetic device as recited in claim 1 appended hereto. Also described is a prosthetic device for use with a hip replacement prosthesis that includes an acetabular cup assembly adapted to be fastened to a patient's pelvis and a femoral stem adapted to be fastened to the patient's femur, where the femoral stem includes a ball component at its proximal end received within the acetabular cup assembly to form a ball joint type coupling. The constraining device includes a semiannular augment adapted to be mounted approximate to a rim of an acetabular cup assembly of a hip replacement prosthesis, where the augment assists in improving stability, at least temporarily, of a ball joint type coupling between the acetabular cup assembly and a femoral stem of the hip replacement prosthesis, and, where the semiannular augment is formed from an augment material that is or includes a biologic material, a biologically absorbable material, and/or a combination of biologic and biologically absorbable materials.

Also described is a hip prosthesis that includes: (a) an acetabular cup assembly adapted to be fastened to a patient's pelvis; (b) a femoral stem adapted to be fastened to the patient's femur, the femoral stem including a ball component at its proximal end received within the acetabular cup assembly to form a ball joint type coupling; and (c) a semiannular augment mounted to a distal end of the acetabular cup assembly, adjacent to the ball component, where the semiannular augment assists in stabilizing the ball joint type coupling between the acetabular cup assembly and the femoral stem and, where the semiannular augment is formed from an augment material that is or includes a biologic material, a biologically absorbable material, and/or a combination of biologic and biologically absorbable materials.

In addition a prosthetic constraining kit is described for implantation in proximity to a hip joint that includes a plurality of constraining augments adapted to be individually fastened on an acetabular prosthesis and/or about an acetabular cavity within a hip bone, and that are adapted to be circumferentially positionable about a femoral member (such as a femur and/or a femoral prosthesis), where the constraining augments at least partially define a central aperture allowing the femoral member to extend therethrough, and where the constraining augments allow a range of angular motion of the femoral member while inhibiting a femoral head of the femoral member from completely passing distally through the central aperture.

Also described is a constraining device for, at least temporarily, promoting engagement of a prosthetic femoral stem component with a prosthetic acetabular component of a prosthetic hip assembly. The constraining device includes a semiannular segment of material that is or includes a biologic material, a biologically absorbable material, and/or a combination of biologic and biologically absorbable materials.

Also described is a restraining device for, at least temporarily, promoting engagement between a first prosthetic joint component or a first bone component and a second prosthetic joint component or a second bone component. The restraining device includes a restraining material that is a biologic material, a biologically absorbable material, and/or a combination of biologic and biologically absorbable materials, and the restraining device does not circumscribe the first prosthetic joint component, the second prosthetic joint component, the first bone component, and/or the second bone component.

A further aspect of the present invention is to provide a method for providing at least temporary stability to a prosthetic hip device, wherein the method provides the prosthetic constraining device of the invention and which includes an acetabular cup assembly for bonding to a patient's pelvis and a femoral stem for bonding to the patient's femur, where the femoral stem includes a ball component at its proximal end received within the acetabular cup assembly to form a ball joint coupling, the method comprising the steps of mounting a plurality of integral snap clip fasteners of stability enhancement snap-on augments to the prosthetic device to improve, in use, at least temporarily, the stability of a joint within the human body wherein each stability enhancing snap-on augment is mountable at selected circumferential positions around the femoral stem, with at least two of said snap-on augments being spaced apart to define a discontinuous ring around the femoral stem, wherein the stability enhancing snap-on enhancement augments are formed from biologically reabsorbable material.

Also described is a method for providing at least temporary stability to a prosthetic hip joint which includes an acetabular cup assembly bonded to a patient's pelvis and a femoral stem bonded to the patient's femur, where the femoral stem includes a ball component at its proximal end received within the acetabular cup assembly to form a ball joint type coupling. The method including the step of mounting a plurality of individual constraining augments on an acetabular prosthesis, where the individual constraining augments at least partially define a central aperture for allowing a femoral component to extend therethrough while inhibiting a femoral head of the femoral component from completely passing distally through the central aperture.

### BRIEF DESCRIPTIONS OF THE DRAWINGS

FIG. 1 is a perspective view, from below, of a prosthetic hip assembly for use in total hip arthroplasty;

FIG. 2 is a cross-sectional schematic view of a prosthetic hip assembly incorporating a permanent constraining ring;

FIG. 3 is an overhead view of an acetabular cup assembly incorporating an exemplary embodiment of the present invention;

FIG. 4 is a cross-sectional, exploded view of a prosthetic hip assembly incorporating an exemplary embodiment of the present invention;

FIG. 5 is a cross-sectional view of a prosthetic acetabular component incorporating another exemplary embodiment of the present invention;

FIG. 6 is an overhead view of a prosthetic acetabular cup component incorporating an additional exemplary embodiment of the present invention;

FIG. 7 is a cross-sectional, exploded view of a prosthetic acetabular cup assembly incorporating an additional exemplary embodiment of the present invention;

FIG. 8 is a cross sectional view of a plurality of exemplary embodiments of the present invention adapted to be mounted to a prosthetic hip assembly; and

FIG. 9 is an overhead view of a prosthetic acetabular cup component incorporating another exemplary embodiment of the present invention.

### DESCRIPTION OF THE PRESENT INVENTION

The examples described herein relate to stabilizing devices and techniques for use in joint reconstruction and replacement surgery. The examples herein will be described with respect to hip reconstruction and total hip arthroplasty, however, it should be understood that these exemplary applications do not limit the scope of the invention to such applications. Thus, it will become apparent to those of ordinary skill in the art that the devices and techniques disclosed herein may be useful for other types of implants and orthopedic surgeries.

For the purposes of this disclosure, "biologic material" refers to material being derived or synthesized from living organisms, cell, tissues, and/or their products. A biologic material may or may not be a biologically reabsorbable material.

For the purposes of this disclosure, "biologically reabsorbable material" refers to material that is biologically compatible with the mammalian body having the capacity to degrade within, dissolve within, and/or be absorbed by the mammalian body. A biologically reabsorbable material may be a biologic material, a naturally occurring material, or a synthetic material.

For the purposes of this disclosure, "biologically acceptable material" refers to material that is biologically compatible with the mammalian body that may not necessarily degrade within, dissolve within, and/or be absorbed by the mammalian body. A biologically acceptable material may be, for example, a biologic material, a biologically reabsorbable material, a naturally occurring material or a synthetic material.

For the purposes of this disclosure, the numeral **"26"** will be used to refer generally to all embodiments of the augments discussed herein. Specific embodiments will be referred to as **"26a", "26b",** etc.

Generally described are biologically reabsorable insert augments that provide initial stability in selected circumferential portions of the acetabular cup, but which may be absorbed over time as the patient's muscles and ligaments heal. Insert augments comprising biologically acceptable material may also be utilized in place of, or in combination with the biologic or biologically reabsorbable augments if desired but are not the subject of the present invention. The augments may be placed anteriorly with respect to the acetabular cup assembly, for example, to mitigate anterior dislocation when the hip is externally rotated and extended. These augments may also or alternatively be located posteriorly to mitigate posterior dislocation when the hip is flexed and internally rotated. Such augments may be semiannular augments, and may snap into place along desired circumferential portions of the rim of the acetabular cup assembly and allow multiple semiannular augments to be utilized in cases of multidirectional instability. Additionally, areas may be left unaugmented along the rim to reduce femoral neck impingement with the acetabular cup assembly with certain angular movements of the femoral stem, and to, in turn, optimize immediate postop range of movement (ROM) and stability. Advantageously, the augments may be mounted to the rims of non-polyethylene hard-bearing inserts of the acetablular cup assembly that often cannot utilize conventional elevations and lips due to impingement and subsequent accelerated wear concerns (from third body particulate debris).

Also described is a system of modular, "snap-on" augments that provide initial stability (constrainment and/or restrainment) along the rim of the acetabular cup and/or insert bearing in the locations desired. In the present invention, such semiannular augments are biologically reabsorbable. Although use of biologic, permanent, and/or combinations of such are also described.

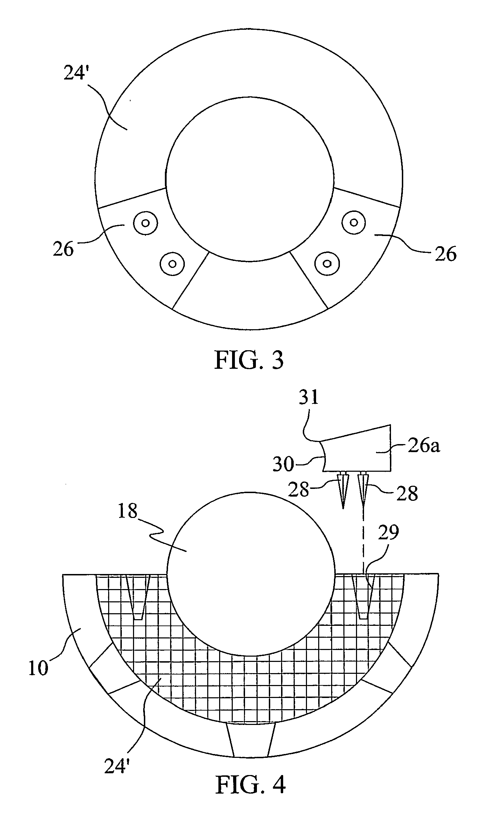

As shown in FIGS. 3 and 4, a bearing insert **24'** according to an exemplary embodiment of the present invention includes a pair of semiannular augments **26a** attached thereto along certain circumferential regions of the distal face of its rim. FIG. 4 provides a cross-sectional, exploded, side-view of the exemplary embodiment shown in FIG. 3, which also displays the ball component **18** of the femoral member and the bearing insert **24'** seated within the acetabular cup **10.**

Referring specifically to FIG. 4, the snap-on semiannular augments **26a** may be attached at two places circumferentially around the rim of the insert bearing **24'** using male couplings **28** that are received within and coupled to female couplings **29** provided in the distal face of the insert bearing **24'** rim. The male couplings **28** may be snap-on mechanisms such as resilient cones (as illustrated) that collapse when being inserted into a narrowed opening (annular shoulder) in the female indentation **29** and expand again when passing the shoulder to be retained within the indentation **29** by the shoulder. Screw mechanisms, snaps, clips, keyways, dowels, adhesives and rivets may also be utilized.

The snap-on semiannular augments **26** in this exemplary embodiment include a semi-spherical shaped inner-radial side surface **30** to provide a radially-inwardly extending lip **31** that acts to constrain the ball component **18** of the femoral member within the acetabular cup assembly.

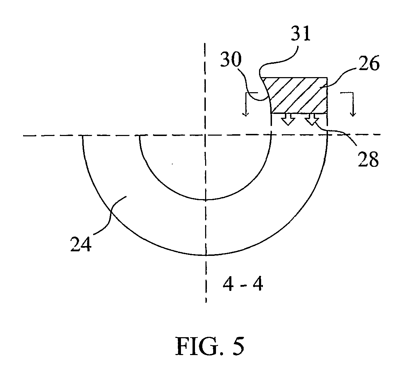

As shown in FIG. 5, axis 4-4 shows the range of movement/constrainment available to the femoral component **14.** In the upper right quadrant, the femoral ball is constrained by the augment **26** to help maintain the ball-joint coupling with the acetabular cup **10,** but range of angular motion of the femoral stem to this quadrant is simultaneously limited by this augment **26.** Conversely, in the upper-left quadrant, the femoral ball is not constrained, and in turn, the range of angular movement of the femoral stem to this quadrant is not as limited. Therefore, the total range of movement available to the patient's femoral component **14** need not be impaired in all 360° about the rim of the acetabular cup assembly to maintain the ball-joint coupling **18** within the acetabular cup/shell **10.**

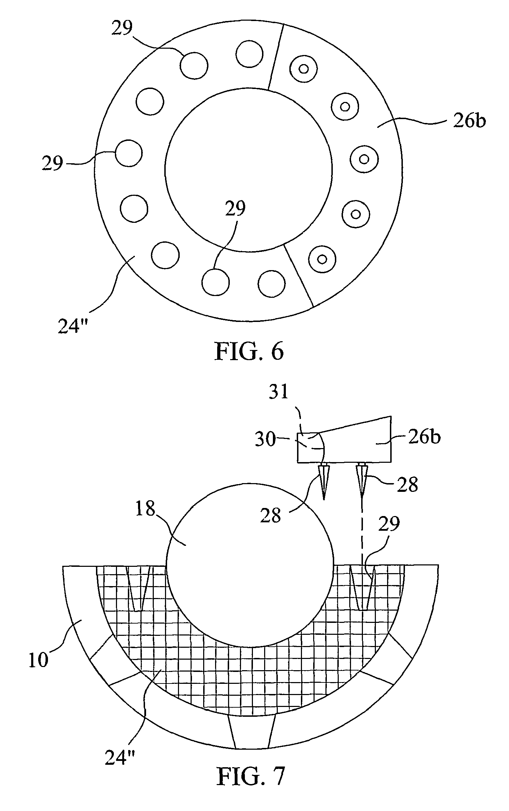

As shown in FIGS. 6 and 7, the snap-on semiannular augment **26b** may be attached at various places circumferentially around the insert bearing **24"** using male couplings **28** that are received within and coupled to female couplings **29** provided in the distal face of the insert bearing **24".** Herein, the female couplings **29** are distributed uniformly about the distal face of the bearing to allow the augment **26b** to be selectively place at various desired positions.

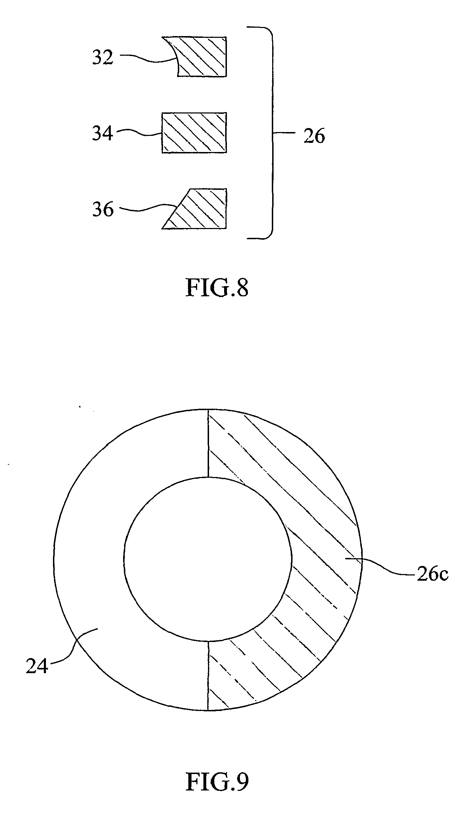

FIG. 8 illustrates exemplary shapes **(32, 34, 36)** of the snap-on semiannular augments **26.** Shape **32** is primarily for constraining purposes, while shapes **34** and **36** are primarily for restraining purposes.

The snap-on semiannular augments **26** can have several different arcuate lengths (compare FIGS. 3, 5 & 9, for example, and without limitation); and can also have several cross-sectional profiles of varying shapes and dimensions (see FIG. 8, for example, and without limitation) depending upon the type and extent of constraint/restraint desired.

FIG. 9 illustrates biologically absorbable snap-on augments **26c** having an arcuate length approximately half of the circumference of the acetabular insert **24.** It will also be appreciated that not all augments need to be semiannular in shape.

The snap-on semiannular augments **26** may be formed from a biologically acceptable material that inhibits corrosion and attack from the patient's body while continuing to provide stability to the hip joint. Beneficially, the snap-on augments **26** and snap clips **28** of the present invention are manufactured from biologically reabsorbable materials that degrade or are absorbed over time as they become nonstructural. Such biologic snap-on augments **26** also allow multiple augments to be snapped into place at multiple segments along the rim in cases of multidirectional instability, with other areas along the rim left un-augmented. This helps prevent impingement of the femoral component **14** in the un-augmented areas while helping optimize immediate postoperative range of movement and stability. Of course, the snap-on semiannular augments **26** may include a combination of biologically acceptable, biologic, and biologically reabsorbable materials as desired.

The biologically reabsorbable snap-on augments **26,** could be formulated to absorbed over a relatively short period (i.e., several weeks or months) and could also be formulated so as to be replaced by tissue (such as scar-tissue) that would provide for long-term hip stability and, hopefully, normal motion. Such formulations of biologic materials are well known by those of ordinary skill in the art.

As will be apparent to those of ordinary skill in the art there are many other biologic and/or biologically reabsorbable materials that can be used for the snap-on augments **26** or snap clips **28,** and there are also new biologic materials being developed on a consistent basis, all of which fall within the scope of the invention.

Examples of biologic materials for use with the snap-on augments **26** and the male fasteners **28** include, without limitation, extra cellular matrices (ECMs). Examples of ECMs include, without limitation, porcine small intestine submucosa (SIS), xenogeneic small intestine submucosa (xSIS), urinary bladder submucosa (UBS), laminated intestinal submucosa, glutaraldehyde-treated bovine pericardium (GLBP). The biologic materials may be layered, molded, formed, braided, perforated, multilaminated, grafted or otherwise manipulated to achieve the desired properties and dimensions associated with the snap-on augments **26** and the male fasteners **28.**

Examples of biologically reabsorbable materials for use with the snap-on augments **26** and the male fasteners **28** include, without limitation, MONOCRYL (poliglecaprone 25), PDS II (polydioxanone), surgical gut suture (SGS), gut, coated VICRYL (polyglactin 910, polyglactin 910 braided), human autograft tendon material, collagen fiber, POLYSORB, poly-L-lactic acid (PLLA), polylactic acid (PLA), polylactides (Pla), racemic form of polylactide (D,L-Pla), poly(L-lactide-coD,L-lactide), 70/30 poly(L-lactide-co-D,L-lactide), polyglycolides (PGa), polyglycolic acid (PGA), polycaprolactone (PCL), polydioxanone (PDS), polyhydroxyacids, and resorbable plate material (see e.g. Orthopedics, October 2002, Vol. 25, No. 10/Supp.). The biologically reabsorbable materials may be layered, molded, formed, braided, perforated, multilaminated, grafted or otherwise manipulated to achieve the desired properties and dimensions associated with the snap-on augments **26** and the male fasteners **28.** For example, the MONOCRYL (poliglecaprone 25), PDS II (polydioxanone), and resorbable plate materials may be block formed, while the surgical gut suture (SGS), gut, coated VICRYL (polyglactin 910), human autograft tendon material, collagen fiber, POLYSORB, poly-L-lactic acid (PLLA), polylactic acid (PLA), polyglycolic acid, and porcine small intestinal submucosa (SIS) material may be layered and formed. Any of the above materials and techniques may be used individually, alternatively, or in conjunction to produce the snap-on augments **26** and the male fasteners **28.**

Also described is to "load" (disburse, coat, impregnate, etc.) the biologically reabsorbable materials comprising the snap-on augments **26** and the male fasteners **28** with agents that could hasten or assist in tissue development, assist in clotting, and/or fight infection. Exemplary agents may include, for example, without limitation, concentrated platelets (SYMPHONY from Depuy Orthapedic) and gentamicin.

Growth stimulating factors may be incorporated into the biologically reabsorbable materials. Examples of such growth stimulating factors include, without limitation, growth factor beta (GFB-β), basic fibroblast growth factor (bFGF), fibroblast growth factor (FGF), epidermal growth factor (EFG), transforming growth factor-β1 (TGF-β1), vascular endothelial growth factor (VEGF), connective tissue growth factor (CTGF), platelet-derived growth factor (PDGF), direct-mediated gene transfer, fibroblast-mediated gene transfer, myoblast-mediated gene transfer, TGF-β gene family, adenovirus-mediated gene transfer, recombinant adenovirus-induced tendon adhesion formation, BMP-12, bone morphogenetic protein-2 gene transfer, growth and differentiation factor-5 (GDF-5) and, insulin like growth factor (IFG). (See e.g. Koski et al., "Tissue-Engineered Ligament---Cells, Matrix, and Growth Factors", July 2000 Tissue Engineering in Orthopedic Surgery, Volume 31, No. 3), (see e.g., Boyer, "Using Growth Factors to Enhance Tendon and Ligament Repair", Orthopaedic Research Society Symposia, AAOS Annual Meeting New Orleans February 2003). Several of these growth factors have been proposed as possible mitogens in fibroblast growth.

Connective tissue stem cells and progenitors may be incorporated with the biologically reabsorbable materials disclosed herein. These connective tissue stem cells and progenitors may be incorporated into the snap-on augments **26** and the male fasteners **28** to provide a three dimensional framework for the creation of engineered tissue. Examples of such connective tissue stem cells and progenitors include, without limitation, fibroblastic colony-forming cells, fibroplast colony-forming units (CFU-F), bone marrow stromal cells, mesenchymal stem cells (MSC), and vascular pericytes. (See e.g. Meschler et al. "Connective Tissue Progenitors: Practical Concepts for Clinical Applications", 2002 Clinical Orthopaedics and Related Research, No. 395, pp. 66-80).

Also envisaged is the incorporation of hematopoietic stem cells and progenitors with the biologic or biologically reabsorbable materials disclosed in the above embodiments. These hematopoietic stem cells and progenitors may be incorporated into the snap-on augments **26** and the male fasteners **28** to provide any cell making up circulating blood and the immune system for, in an exemplary application, inhibiting infection after surgery.

## Claims

1. A prosthetic constraining device for mounting on or approximate to a hip joint within the human body to temporarily bolster the stability to the hip joint; **characterized in that** the constraining device comprises
an acetabular insert (24) having mating features to engage corresponding mating features of an acetabular cup (10) adapted to be permanently mounted to the patient's pelvis; and
a semiannular augment (26) mountable on the acetabular insert (24) wherein the augment (26) is formed from biologically reabsorbable material and assists in multidirectional stability, at least temporarily, of a ball joint type coupling;
**characterised in that** the constraining device further comprises at least one additional semiannular augment (26), wherein the semiannular augments are snap-on augments and
each of the augments (26) includes at least one integrated reabsorbable snap clip fastener (28) whereby at least two of said augments may be spaced apart at selected circumferential positions to define a discontinuous ring on the acetabular insert (24) such as to reduce impingement.

2. The prosthetic constraining device of claim 1, also comprising at least one of:
a screw;
a snap;
a clip;
a keyway;
a dowel; and
a rivet.

3. The prosthetic constraining device of claim 1, wherein the semiannular snap-on augments (26) are formed from at least one of:
porcine small intestine submucosa (SIS);
xenogeneic small intestine submucosa (xSIS);
urinary bladder submucosa (UBS);
laminated intestinal submucosa; and
glutaraldehyde-treated bovine pericardium (GLBP).

4. The prosthetic constraining device of claim 1, wherein a surface of each of the plurality of semiannular snap-on augments (26) is contoured to approximate the shape of a portion of a neck of a femoral prosthesis potentially coming into contact therewith.

5. The prosthetic constraining device of claim 1, wherein the plurality of semiannular snap-on augments (26) are positioned on an anterior/superior portion of the rim of the acetabular cup assembly.

6. The prosthetic constraining device of claim 1, wherein:
the plurality of semiannular snap-on augments (26) include a contoured, radially inner surface to approximate an outer surface of the ball of a femoral prosthesis potentially coming into contact therewith.

7. The prosthetic constraining device of claim 6, wherein the contoured, radially inner surface of each snap-on augment (26) is substantially semi-spherically shaped.

8. The prosthetic constraining device of claim 6, wherein the contoured, radially inner surface is substantially arcuate.

9. The prosthetic constraining device of claim 1, wherein the plurality of semiannular snap-on augments (26) are mounted to a proximal rim surface of the acetabular cup assembly.

10. The prosthetic constraining device of claim 1, wherein the plurality of semiannular snap-on augments (26) are mounted to a proximal rim surface of a cup-shaped bearing insert (24) component of the acetabular prosthesis.

11. The prosthetic constraining device of claim 1, wherein the at least one integrated snap clip fastener includes a snap-on retention member enabling snap-on-type mounting of the plurality of semiannular snap-on augments (26) to the acetabular cup assembly (10).

12. The prosthetic constraining device of claim 1, wherein the augment material is supplemented with a clotting agent.

13. The prosthetic constraining device of claim 1, wherein the augment material is supplemented with an antibacterial agent.

14. The prosthetic constraining device of claim 1, wherein the semiannular snap-on augments (26) are applied in the form of a paste material.

15. An *ex vivo* method for providing at least temporary stability to a prosthetic hip device, wherein the method provides the prosthetic constraining device of claim 1 which includes an acetabular cup assembly (10) for bonding to a patient's pelvis and a femoral stem (14) for bonding to the patient's femur, where the femoral stem includes a ball component at its proximal end received within the acetabular cup assembly (10) to form a ball joint coupling (18), the method comprising the step of:
mounting a plurality of integral snap clip fasteners (28) of stability enhancement snap on augments (26) to the prosthetic device to improve, in use, at least temporarily, the stability of a joint within a human body, wherein each stability enhancing snap on augment is mountable at selected circumferential positions around the femoral stem, with at least two of said snap on augments being spaced apart to define a discontinuous ring around the femoral stem, wherein the stability enhancement snap on augments (26) are formed from biologically reabsorbable material.

16. The method of claim 15, wherein:
the mounting step includes the step of mounting the stability enhancement snap-on augments (26) about a rim of an acetabular cup assembly (10) where, in use, a femoral stem passes through the aperture.

17. The method of claim 15 or 16, wherein the snap clip integral fasteners (28) comprise at least one of:
a screw;
a snap;
a clip;
a keyway;
a dowel; and
a rivet.

18. The method of claim 15 or 16, wherein the stability enhancement snap-on augments (26) are in the form of a paste material and the mounting step includes the step of applying the paste material such that in use it is in proximity to the joint.

19. The method of claim 15, wherein the augment material is loaded with at least one of a clotting agent and an antibacterial agent.

## Patentansprüche

1. Eine prothetische Beschränkungsvorrichtung zum Befestigen an oder nahe einem Hüftgelenk innerhalb des menschlichen Körpers, um die Stabilität des Hüftgelenks vorübergehend zu stärken; **dadurch gekennzeichnet, dass** die Beschränkungsvorrichtung Folgendes beinhaltet:
einen Hüftpfanneneinsatz (24) mit zusammenpassenden Strukturen, um in entsprechende zusammenpassende Strukturen einer Hüftpfannenschale (10) einzugreifen, die angepasst ist, um permanent an dem Becken des Patienten befestigt zu werden; und
eine halbringförmige Vergrößerung (26), die an dem Hüftpfanneneinsatz (24) befestigt werden kann, wobei die Vergrößerung (26) aus einem biologisch reabsorbierbaren Material gebildet ist und die multidirektionale Stabilität einer kugelgelenkartigen Kopplung mindestens vorübergehend unterstützt;
**dadurch gekennzeichnet, dass** die Beschränkungsvorrichtung ferner mindestens eine zusätzliche halbringförmige Vergrößerung (26) beinhaltet, wobei die halbringförmigen Vergrößerungen aufschnappbare Vergrößerungen sind, und jede der Vergrößerungen (26) mindestens ein integriertes reabsorbierbares Schnappklemmenbefestigungsmittel (28) umfasst, wobei mindestens zwei der Vergrößerungen an ausgewählten Umfangspositionen mit Abstand voneinander angeordnet sein können, um einen diskontinuierlichen Ring auf dem Hüftpfanneneinsatz (24) zu definieren, um so Impingement zu reduzieren.

2. Prothetische Beschränkungsvorrichtung gemäß Anspruch 1, die außerdem mindestens eines von Folgendem beinhaltet:
eine Schraube;
einen Schnappverschluss;
eine Klemme;
eine Keilnut;
einen Stift; und
einen Niet.

3. Prothetische Beschränkungsvorrichtung gemäß Anspruch 1, wobei die halbringförmigen aufschnappbaren Vergrößerungen (26) aus mindestens einem von Folgendem gebildet sind:
Schweinedünndarmsubmukosa (SIS);
xenogener Dünndarmsubmukosa (xSIS);
Harnblasensubmukosa (UBS);
lamellärer Darmsubmukosa; und
mit Glutaraldehyd behandeltem Rinderherzbeutel (GLBP).

4. Prothetische Beschränkungsvorrichtung gemäß Anspruch 1, wobei eine Oberfläche jeder der Vielzahl von halbringförmigen aufschnappbaren Vergrößerungen (26) konturiert ist, um der Form eines Abschnitts eines Halses einer Femurprothese, der möglicherweise mit ihr in Kontakt kommt, zu ähneln.

5. Prothetische Beschränkungsvorrichtung gemäß Anspruch 1, wobei die Vielzahl von halbringförmigen aufschnappbaren Vergrößerungen (26) an einem anterioren/superioren Abschnitt des Randes der Hüftpfannenschalenanordnung positioniert ist.

6. Prothetische Beschränkungsvorrichtung gemäß Anspruch 1, wobei:
die Vielzahl von halbringförmigen aufschnappbaren Vergrößerungen (26) eine konturierte, radial innere Oberfläche umfasst, um einer äußeren Oberfläche der Kugel einer Femurprothese, die möglicherweise mit ihr in Kontakt kommt, zu ähneln.

7. Prothetische Beschränkungsvorrichtung gemäß Anspruch 6, wobei die konturierte, radial innere Oberfläche jeder aufschnappbaren Vergrößerung (26) im Wesentlichen halbkugelförmig geformt ist.

8. Prothetische Beschränkungsvorrichtung gemäß Anspruch 6, wobei die konturierte, radial innere Oberfläche im Wesentlichen bogenförmig ist.

9. Prothetische Beschränkungsvorrichtung gemäß Anspruch 1, wobei die Vielzahl von halbringförmigen aufschnappbaren Vergrößerungen (26) an einer proximalen Randoberfläche der Hüftpfannenschalenanordnung befestigt ist.

10. Prothetische Beschränkungsvorrichtung gemäß Anspruch 1, wobei die Vielzahl von halbringförmigen aufschnappbaren Vergrößerungen (26) an einer proximalen Randoberfläche einer schalenförmigen Lagereinsatz(24)-Komponente der Hüftpfannenprothese befestigt ist.

11. Prothetische Beschränkungsvorrichtung gemäß Anspruch 1, wobei das mindestens eine integrierte Schnappklemmenbefestigungsmittel ein aufschnappbares Rückhalteelement umfasst, das eine aufschnappartige Befestigung der Vielzahl von halbringförmigen aufschnappbaren Vergrößerungen (26) an der Hüftpfannenschalenanordnung (10) ermöglicht.

12. Prothetische Beschränkungsvorrichtung gemäß Anspruch 1, wobei das Vergrößerungsmaterial mit einem Gerinnungswirkstoff ergänzt ist.

13. Prothetische Beschränkungsvorrichtung gemäß Anspruch 1, wobei das Vergrößerungsmaterial mit einem antibakteriellen Wirkstoff ergänzt ist.

14. Prothetische Beschränkungsvorrichtung gemäß Anspruch 1, wobei die halbringförmigen aufschnappbaren Vergrößerungen (26) in der Form eines Pastenmaterials angewendet werden.

15. Ein *Ex-vivo*-Verfahren zum Bereitstellen mindestens einer vorübergehenden Stabilität für eine prothetische Hüftvorrichtung, wobei das Verfahren die prothetische Beschränkungsvorrichtung gemäß Anspruch 1 bereitstellt, die eine Hüftpfannenschalenanordnung (10) zum Verbinden mit dem Becken eines Patienten und einen Femurstamm (14) zum Verbinden mit dem Femur des Patienten umfasst, wobei der Femurstamm an seinem proximalen Ende eine Kugelkomponente umfasst, die innerhalb der Hüftpfannenschalenanordnung (10) aufgenommen wird, um eine Kugelgelenkkopplung (18) zu bilden, wobei das Verfahren den folgenden Schritt beinhaltet:
Befestigen einer Vielzahl von integralen Schnappklemmenbefestigungsmitteln (28) von aufschnappbaren Stabilitätssteigerungsvergrößerungen (26) an der prothetischen Vorrichtung, um bei Gebrauch mindestens vorübergehend die Stabilität eines Gelenks innerhalb eines menschlichen Körpers zu verbessern, wobei jede aufschnappbare stabilitätssteigernde Vergrößerung an ausgewählten Umfangspositionen um den Femurstamm befestigt werden kann, wobei mindestens zwei der aufschnappbaren Vergrößerungen voneinander mit Abstand angeordnet sind, um einen diskontinuierlichen Ring um den Femurstamm zu definieren, wobei die aufschnappbaren Stabilitätssteigerungsvergrößerungen (26) aus biologisch reabsorbierbarem Material gebildet sind.

16. Verfahren gemäß Anspruch 15, wobei:
der Befestigungsschritt den Schritt des Befestigens der aufschnappbaren Stabilitätssteigerungsvergrößerungen (26) um einen Rand einer Hüftpfannenschalenanordnung (10) umfasst, wo bei Gebrauch ein Femurstamm durch die Öffnung geht.

17. Verfahren gemäß Anspruch 15 oder 16, wobei die integralen Schnappklemmenbefestigungsmittel (28) mindestens eines von Folgendem beinhalten:
eine Schraube;
einen Schnappverschluss;
eine Klemme;
eine Keilnut;
einen Stift; und
einen Niet.

18. Verfahren gemäß Anspruch 15 oder 16, wobei die aufschnappbaren Stabilitätssteigerungsvergrößerungen (26) in der Form eines Pastenmaterials vorliegen und der Befestigungsschritt den Schritt des Anwendens des Pastenmaterials, so dass es sich bei Gebrauch in der Nähe des Gelenks befindet, umfasst.

19. Verfahren gemäß Anspruch 15, wobei das Vergrößerungsmaterial mit mindestens einem von einem Gerinnungswirkstoff und einem antibakteriellen Wirkstoff beladen ist.

## Revendications

1. Un dispositif de contrainte prothétique destiné à se monter sur ou à proximité d'une articulation de la hanche à l'intérieur du corps humain pour renforcer de manière temporaire la stabilité de l'articulation de la hanche ; **caractérisé en ce que** le dispositif de contrainte comprend
un insert acétabulaire (24) présentant des caractéristiques d'accouplement pour se mettre en prise avec des caractéristiques d'accouplement correspondantes d'une cupule acétabulaire (10) conçue pour être montée de façon permanente sur le bassin du patient ; et
un rajout semi-annulaire (26) pouvant être monté sur l'insert acétabulaire (24), le rajout (26) étant formé à partir de matière biologiquement résorbable et contribuant à la stabilité multidirectionnelle, au moins de manière temporaire, d'un couplage de type articulation à rotule ;
**caractérisé en ce que** le dispositif de contrainte comprend en outre au moins un rajout semi-annulaire (26) supplémentaire, les rajouts semi-annulaires étant des rajouts encliquetables et chacun des rajouts (26) incluant au moins une attache formant clip d'encliquetage résorbable solidarisée (28) grâce à quoi au moins deux desdits rajouts peuvent être espacés à l'écart l'un de l'autre à des positions circonférentielles sélectionnées afin de définir un anneau discontinu sur l'insert acétabulaire (24) de manière à réduire un coincement.

2. Le dispositif de contrainte prothétique de la revendication 1, comprenant également au moins :
soit une vis ;
soit un encliquetage ;
soit un clip ;
soit une rainure de clavette ;
soit un tenon ;
soit un rivet.

3. Le dispositif de contrainte prothétique de la revendication 1, dans lequel les rajouts encliquetables semi-annulaires (26) sont formés à partir d'au moins :
soit une sous-muqueuse d'intestin grêle de porc (SIS) ;
soit une sous-muqueuse d'intestin grêle xénogénique (xSIS) ;
soit une sous-muqueuse de vessie urinaire (UBS) ;
soit une sous-muqueuse intestinale stratifiée ;
soit un péricarde bovin traité au glutaraldéhyde (GLBP).

4. Le dispositif de contrainte prothétique de la revendication 1, dans lequel une surface de chaque rajout de la pluralité de rajouts encliquetables semi-annulaires (26) est profilée afin de s'approcher de la forme d'une portion d'un col d'une prothèse fémorale venant potentiellement à son contact.

5. Le dispositif de contrainte prothétique de la revendication 1, dans lequel la pluralité de rajouts encliquetables semi-annulaires (26) sont positionnés sur une portion antérieure / supérieure du rebord de l'ensemble formant cupule acétabulaire.

6. Le dispositif de contrainte prothétique de la revendication 1, dans lequel :
la pluralité de rajouts encliquetables semi-annulaires (26) incluent une surface radialement interne profilée afin de s'approcher d'une surface externe de la rotule d'une prothèse fémorale venant potentiellement à son contact.

7. Le dispositif de contrainte prothétique de la revendication 6, dans lequel la surface radialement interne profilée de chaque rajout encliquetable (26) est de forme substantiellement semi-sphérique.

8. Le dispositif de contrainte prothétique de la revendication 6, dans lequel la surface radialement interne profilée est substantiellement arquée.

9. Le dispositif de contrainte prothétique de la revendication 1, dans lequel la pluralité de rajouts encliquetables semi-annulaires (26) sont montés sur une surface de rebord proximale de l'ensemble formant cupule acétabulaire.

10. Le dispositif de contrainte prothétique de la revendication 1, dans lequel la pluralité de rajouts encliquetables semi-annulaires (26) sont montés sur une surface de rebord proximale d'un composant d'insert porteur en forme de cupule (24) de la prothèse acétabulaire.

11. Le dispositif de contrainte prothétique de la revendication 1, dans lequel l'au moins une attache formant clip d'encliquetage solidarisée inclut un élément de retenue encliquetable permettant un montage de type encliquetable de la pluralité de rajouts encliquetables semi-annulaires (26) sur l'ensemble formant cupule acétabulaire (10).

12. Le dispositif de contrainte prothétique de la revendication 1, dans lequel la matière du rajout est additionnée d'un agent de coagulation.

13. Le dispositif de contrainte prothétique de la revendication 1, dans lequel la matière du rajout est additionnée d'un agent antibactérien.

14. Le dispositif de contrainte prothétique de la revendication 1, dans lequel les rajouts encliquetables semi-annulaires (26) sont appliqués sous la forme d'une matière pâteuse.

15. Une méthode ex vivo pour fournir une stabilité au moins temporaire à un dispositif de hanche prothétique, la méthode fournissant le dispositif de contrainte prothétique de la revendication 1, lequel inclut un ensemble formant cupule acétabulaire (10) destiné à se fixer au bassin d'un patient et une tige fémorale (14) destinée à se fixer au fémur du patient, la tige fémorale incluant un composant de rotule au niveau de son extrémité proximale reçu au sein de l'ensemble formant cupule acétabulaire (10) afin de former un couplage d'articulation à rotule (18), la méthode comprenant l'étape consistant à :
monter une pluralité d'attaches formant clips d'encliquetage solidaires (28) de rajouts encliquetables d'augmentation de stabilité (26) sur le dispositif prothétique afin d'améliorer, lors de l'utilisation, au moins de manière temporaire, la stabilité d'une articulation à l'intérieur d'un corps humain, chaque rajout encliquetable augmentant la stabilité pouvant être monté à des positions circonférentielles sélectionnées autour de la tige fémorale, au moins deux desdits rajouts encliquetables étant espacés l'un de l'autre afin de définir un anneau discontinu autour de la tige fémorale, les rajouts encliquetables d'augmentation de stabilité (26) étant formés à partir de matière biologiquement résorbable.

16. La méthode de la revendication 15, dans laquelle :
l'étape de montage inclut l'étape consistant à monter les rajouts encliquetables d'augmentation de stabilité (26) autour d'un rebord d'un ensemble formant cupule acétabulaire (10) où, lors de l'utilisation, une tige fémorale passe au travers de l'ouverture.

17. La méthode de la revendication 15 ou de la revendication 16, dans laquelle les attaches solidaires formant clips d'encliquetage (28) comprennent au moins :
soit une vis ;
soit un encliquetage ;
soit un clip ;
soit une rainure de clavette ;
soit un tenon ;
soit un rivet.

18. La méthode de la revendication 15 ou de la revendication 16, dans laquelle les rajouts encliquetables d'augmentation de stabilité (26) sont sous la forme d'une matière pâteuse et l'étape de montage inclut l'étape consistant à appliquer la matière pâteuse de telle sorte que, lors de l'utilisation, elle se trouve à proximité de l'articulation.

19. La méthode de la revendication 15, dans laquelle la matière du rajout est chargée d'au moins soit un agent de coagulation, soit un agent antibactérien.
